# EUROPEAN PATENT SPECIFICATION

(11) **EP 1 759 699 B1**
(45) Date of publication and mention of the grant of the patent: **20.01.2016**
(21) Application number: 05751499.4
(22) Date of filing: 17.06.2005
(51) Int. Cl.: A61K 31/4745, A61K 9/127, A61K 47/12, A61K 47/24, A61K 47/28, A61P 35/00

(54) **LIPOSOME PREPARATION CONTAINING SLIGHTLY WATER-SOLUBLE CAMPTOTHECIN**
LIPOSOMZUBEREITUNG MIT LEICHT WASSERLÖSLICHEM CAMPTOTHECIN
PRÉPARATION DE LIPOSOME CONTENANT DU CAMPTOTHECIN LEGÉREMENT SOLUBLE DANS L'EAU

(30) Priority: 18.06.2004 JP 2004181408
(43) Date of publication of application: 07.03.2007
(73) Proprietor: Kabushiki Kaisha Yakult Honsha, Tokyo 105-8660 (JP)
(72) Inventor: YOSHINO, Keisuke, Ashigarakami-gun, Kanagawa 2590151 (JP); NOZAWA, Shigenori, Ashigarakami-gun, Kanagawa 2590151 (JP); OGATA, Yoshitaka, Ashigarakami-gun, Kanagawa 2590151 (JP); KUROSAKI, Yasuo, Ashigarakami-gun, Kanagawa 2590151 (JP); SAWADA, Seigo, Tokyo 1058660 (JP); KATO, Ikuo, Tokyo 1058660 (JP); MATSUZAKI, Takeshi, Tokyo 1058660 (JP)
(74) Representative: TBK
(86) International application number: PCT/JP2005/011158
(87) International publication number: WO 2005/123078

(56) References cited:
- EP-A1- 1 190 706
- WO-A1-00/23052
- WO-A2-02/058622
- JP-A- 9 504 517
- JP-A- 60 155 109
- YANG S.C. ET AL.: "Body distribution in mice of intravenoulsy injected camptothecin solid lipid nanoparticles and targeting effect on brain", JOURNAL OF CONTROLLED RELEASE, vol. 59, no. 3, 1999, pages 299-307, XP002673675,
- CROSASSO P. ET AL.: 'Preparation, characterization and properties of sterically stabilized paclitaxel-containing liposomes.' J CONTROL RELEASE vol. 63, no. 1-2, 2000, pages 19 - 30, XP004185068

## Description

### TECHNICAL FIELD

The present invention relates to a liposome preparation especially containing as a drug a slightly water-soluble camptothecin compound.

### BACKGROUND ART

In recent years, the drug delivery system (DDS) has aggressively been investigated by which a drug is safely and efficiently delivered to and distributed in a targeted lesion site. As one method thereof, there has been examined use of liposomes as a transport tool (i.e., carrier) for a drug. Liposomes are closed vesicles provided in the form of an aqueous dispersion, each of which is formed of a lipid bilayer membrane of a phospholipid, and the inner space of each vesicle presents an aqueous environment (i.e., internal aqueous phase). Up to now, various drugs have been investigated for their incorporation into a liposome. In the incorporation of a drug into a liposome, a water-soluble drug is mainly held in the inner space presenting an aqueous environment, while a slightly water-soluble drug is mainly carried and held in lipid membrane layers presenting an oily environment. The liposome membrane has also been investigated, and there has been proposed to incorporate a higher fatty acid, or preferably, oleic acid in the liposome membrane as a membrane component to improve the rate of holding a water-soluble drug, such as urokinase, encapsulated in a liposome vesicle (see Patent Document 1).

Meanwhile, topoisomerase inhibitors belong to one category of pharmaceuticals to be used for cancer treatment, and camptothecin is one example thereof. Camptothecin is the alkaloid of a 5-membered cyclic structure which was extracted and isolated from a Chinese plant Kiju (i.e., Camptotheca acuminata) by Wall et al. of the USA in 1966, and was found to have high antitumor activities and a broad antitumor spectrum (see Non-patent Document 1). Conventional chemotherapeutic agents for cancer express antitumor activities due to inhibition of topoisomerase II. In contrast, camptothecin inhibits topoisomerase I, to thereby inhibit actions of the topoisomerase which plays a role in replication, repair and recombination of DNA, and transcription.

Some camptothecin analog compounds having antitumor activities are also known. Examples thereof include a camptothecin derivative which is camptothecin (see the chemical formula (1) shown below) having a substituent at its 7th position, such as: 7-(β-trimethylsilyl)ethylcamptothecin (see Patent Document 2); 7-(t-butoxy)iminomethylcamptothecin (see Patent Document 3); and 7-ethyl-10-hydroxycamptothecin (SN-38) as an active metabolite (i.e., active body) of irinotecan (CPT-11) obtained by an action of carboxyesterase. Those camptothecin analog compounds themselves are antitumor active species, and are water-insoluble or slightly water-soluble unlike irinotecan which is a water-soluble prodrug. However, α-hydroxylactone rings in molecules of those compounds are sensitive to an aqueous environment, and ring-opening occurs due to hydrolysis in the aqueous environment with high pH, for instance, in blood. When the α-hydroxylactone rings are open, camptothecin and analogue compounds thereof do not exert antitumor activities which are originally to be exerted (see Non-patent Document 2).

The incorporation into a liposome is thought to be an approach to the solution of the problems as above which makes it possible to stably and efficiently deliver camptothecin or a water-insoluble or slightly water-soluble analogue compound thereof (hereinafter, sometimes referred to as "slightly water-soluble camptothecin compound") to a lesion site so that antitumor activities may be expressed in the targeted lesion site.

Some examples of the incorporation of camptothecin into a liposome have been reported, which include a report that shows the hydrolysis of the α-hydroxylactone rings can be suppressed by incorporating camptothecin into a liposome (see, for example, Non-patent Documents 3 and 4 and Patent Document 4).

In the incorporation of camptothecin into a liposome, the slightly water-soluble camptothecin compounds which are not compatible with an aqueous environment are thought to be carried in lipid membrane layers as described above. However, the camptothecin compounds as antitumor active species are known to have a poor compatibility with a lipid membrane even though the camptothecin compounds are considered as lipophilic or lipid-soluble, and to be difficult to stabilize in the membrane. Thus, there arises a problem in that the liposome preparation containing a camptothecin compound is inferior in ability to hold the drug and in retention in blood for the drug. When the liposome preparation is administered into blood, the slightly water-soluble camptothecin compound rapidly leaves a liposome and is released in a blood flow, and rapidly disappears from the blood through adhesion onto a blood vessel wall, or the like, resulting in difficulty in maintaining the concentration of the compound in plasma for a long period of time.

As an example of the incorporation of camptothecin into a liposome, it has been proposed to form a lipid complex with addition of N-glutaryl phosphatidyl ethanolamine (see Patent Document 5 and Non-patent Document 5). It is alleged in the proposals that a large amount of camptothecin which has a poor compatibility with a lipid membrane can be carried. However, the lipid complex disappears from blood at high speed when administered to a mouse, as shown in the proposals.

In addition, a liposomal agent of SN-38 that is the active body of irinotecan has been reported with respect to its retention in blood for the drug. It has been reported that, when the SN-38 liposome was administered to, for example, a mouse, the SN-38 concentration in blood rapidly decreased immediately after the administration, and the SN-38 concentration in plasma was difficult to maintain (see Non-patent Document 6).

There is another report that quantitative analysis of the SN-38 concentration in plasma was performed by using high performance liquid chromatography so as to find that the concentration of SN-38 in the plasma of a dog was difficult to maintain (see Non-patent Document 7).
PATENT DOCUMENT 1: JP 60-155109 A
PATENT DOCUMENT 2: JP 2001-511807 A
PATENT DOCUMENT 3: JP 2002-539128 A
PATENT DOCUMENT 4: JP 09-504517 A
PATENT DOCUMENT 5: US 5,834,012

NON-PATENT DOCUMENT 1: Hans-Georg Lerchen, Drugs Fut, 27(9), 2002, 869-878
NON-PATENT DOCUMENT 2: Am. Chem. Soc., 94, 1966, 388
NON-PATENT DOCUMENT 3: Tomas G. Burke et al., J. Am. Chem. Soc. 114, 1992, 8318-8319
NON-PATENT DOCUMENT 4: Tomas G. Burke et al., Biochemistry, 32, 1993, 5352-5364
NON-PATENT DOCUMENT 5: Cancer Chem. Pharm. 37, 1996, 531-538
NON-PATENT DOCUMENT 6: Joshua Williams et al., Journal of Controlled Release 91, 2003, 167-172
NON-PATENT DOCUMENT 7: W. Guo et al., Journal of Chromatography B, 791, 2003, 85-92

### DISCLOSURE OF THE INVENTION

### PROBLEMS TO BE SOLVED BY THE INVENTION

It is desired to provide a liposome preparation with an excellent retention in blood for a drug, which can stably hold a slightly water-soluble camptothecin compound for a long period of time, whereby hydrolysis of an α-hydroxylactone ring in blood is suppressed and the slightly water-soluble camptothecin compound is kept in a structure in which the ring is not opened and which makes the compound effective as an antitumor-active drug, and, moreover, the liability of the drug to leave a liposome is advantageously reduced, so that it is possible to maintain the drug concentration in plasma for a long period of time after administration.

WO 02/058622 A2 is directed at lipid complexes (e.g. liposomes) of the camptothecin compound SN-38.

JP 60 155109 A is directed at a liposome pharmaceutical being obtained by incorporating about 5 % by weight of a so-called higher fatty acid with a lipid in a liposome for retaining chemical compounds like drugs.

### MEANS TO SOLVE THE PROBLEMS

The inventors of the present invention have examined the drug holding ability and retention in blood of a liposome preparation especially containing a slightly water-soluble camptothecin compound as a drug. As a result, the inventors of the present invention have found that the ability to hold a slightly water-soluble camptothecin compound can significantly be improved and controlled release of the drug can be improved by incorporating a fatty acid into the liposome. This is thought to be because the withdrawal of a slightly water-soluble camptothecin compound is competitively inhibited by the fatty acid on which a protein in blood exerts a withdrawal action based on a similar mechanism, leading to a much improved ability to hold the slightly water-soluble camptothecin compound. The effect of the competitive inhibition is further significant when the amount of the fatty acid in the liposome preparation is larger than that of the drug. Another reason is assumed to be that flexibility is imparted to the membrane of the liposome by incorporating a fatty acid therein and, consequently, the stabilization of the slightly water-soluble camptothecin compound in the membrane is achieved, which compound originally has inferior stability in a lipid membrane, thus resulting in the improvement of the holding property.

Further, it was also found that modification of the membrane surface with a hydrophilic polymer chain, in particular, polyethylene glycol, in the liposome preparation as such contributes to the improvement of the retention in blood. It should be noted that, as a method of increasing the retention in blood by improving the kinetics of a liposome in blood, modification of the membrane surface with a hydrophilic polymer chain is known especially in the case where the drug to be incorporated into a liposome is a water-soluble drug which is essentially held in a vesicle. For a slightly water-soluble drug, the effect of increasing the reteintion in blood can also be obtained as long as the drug is compatible with a lipid membrane and is held by a liposome even in blood. For example, it has been reported that a liposome preparation of Paclitaxel that is a slightly water-soluble drug had significantly (about 5 folds) increased retention in blood by the membrane modification with polyethylene glycol-phosphatidylethanolamine (PEG-PE) (J. of Controlled Release 63, 2000, 19-30). However, in the incorporation of a slightly water-soluble camptothecin compound into a liposome, the improvement in retention in blood due to the PEG-PE modification of the typical membrane of the liposome containing no fatty acids is not remarkable as described later in Comparative Examples. It was confirmed that sufficient effects could not be obtained by such a modification alone in terms of a practical production of the preparation.

In addition, in the case of subjecting a liposome preparation containing a slightly water-soluble camptothecin compound to particle size reduction by employing a diaphragm passage method which is general as the particle sizing method for a liposome, the drug carried by the lipid membrane easily leave the membrane when the liposome preparation passes through a diaphragm. It was found that, especially when an extruder having a small pore diameter of about 300 nm or less is used, the drug tends to leave the membrane at the time of particle sizing. According to those findings, the inventors of the present invention have further investigated and found that, in the case of a liposome preparation especially containing as a drug a slightly water-soluble camptothecin compound, a particle sizing method by which a high emulsification energy can be applied owing to liquid-liquid collision (i.e., shear) between liposome preparations (as dispersions) in the form of a high-pressure jet is preferable for obtaining a liposome preparation having a small particle size and stably holding the drug. Based on those findings, the following are provided according to the present invention so as to solve the above-mentioned problems.
(1) A liposome preparation as defined in claim 1. The slightly water-soluble camptothecin compound is 20(S)-camptothecin or a slightly water-soluble derivative thereof having a substituent at its 7th position, and is specifically exemplified by those mentioned in the following item (2).
(2) The liposome preparation according to the above item (1), in which the slightly water-soluble camptothecin compound includes 20(S)-camptothecin, 7-ethyl-10-hydroxycamptothecin (SN-38), 7-(β-trimethylsilyl)ethylcamptothecin, or 7-(t-butoxy)iminomethylcamptothecin.

In the present invention, a particularly preferable fatty acid is stearic acid.
(3) The liposome preparation according to any one of the above items (1) or (2), in which the phospholipid includes a hydrogenated phospholipid.

Examples of the phospholipid preferably include sphingophospholipid.
(4) The liposome preparation according to any one of the above items (1) to (3), further including as the lipid components at least one other lipid in addition to the phospholipid and the fatty acid.
(5) The liposome preparation according to the above item (4), in which the other lipid includes cholesterol.

In the present invention, a preferable example of composition of the lipid components constituting a membrane is such that the phospholipid is a hydrogenated phospholipid, and cholesterol is included in the lipid components in addition to the hydrogenated phospholipid and the fatty acid.

The polyethylene glycol (PEG) chain generally has a molecular weight of about 500 to 10,000 daltons.

Specific examples of a lipid derivative into which a hydrophilic modification group is introduced include phosphatidylethanolamine ligated with polyethylene glycol (PEG-PE) and cholesterol ligated with polyethylene glycol (Chol-PEG).

In the liposome preparation of the present invention, in particular, the quantitative ratio between a drug and lipid components constituting a membrane, and the quantitative ratio of fatty acids to the entire lipid components are preferably as described below.
(6) The liposome preparation according to any one of the above items (1) to (5), in which the liposome preparation has a drug/lipid molar ratio of 0.001 to 0.1 or less.
(7) The liposome preparation according to any one of the above items (1) to (6), in which the fatty acid is included in a ratio of 1 to 30 mol% of the total mol of the lipid components.

The content of the fatty acid is preferably 5 mol% or more, more preferably 10 mol% or more, and further preferably 12 mol% or more. In addition, the upper limit thereof is preferably 26 mol% or less.
(8) The liposome preparation according to any one of the above items (1) to (7) has an average particle diameter of preferably 50 to 300 nm, more preferably 70 to 200 nm, and further preferably 90 to 150 nm, with an average particle diameter of 140 nm or less being particularly preferable.
(9) The liposome preparation according to any one of the above items (1) to (8), in which the liposome preparation is observed as particles with an irregular, nonspherical shape under transmission electron microscopy after freezing in the presence of glycerin.
(10) A pharmaceutical composition, including the liposome preparation according to any one of the above items (1) to (9). [0025]

### EFFECTS OF THE INVENTION

The liposome preparation of the present invention as described above can stably hold a slightly water-soluble camptothecin compound as a drug in active species form (i.e., antitumor-active drug), and is excellent in retention in blood. Therefore, the liposome preparation is excellent in controlled release of the drug and, after being administered, can suppress hydrolysis of an α-hydroxylactone ring so that the drug is retained in blood for a long period of time in a structural state allowing a drug activity, whereby the concentration of the drug in plasma can be maintained for a long period of time after the administration.

### BRIEF DESCRIPTION OF THE DRAWINGS

[FIG. 1] FIG. 1 is a photographic view showing the image of an SN-38-containing liposome (HSPC:Chol:SA = 7:7:5) of the present invention, which was observed with an electron microscope using freeze-fracture replica technique.
[FIG. 2] FIG. 2 is a photographic view showing the image of an SN-38-containing liposome (HSPC:Chol:SA = 7:7:0) prepared in Comparative Example 1, which was observed with an electron microscope using freeze-fracture replica technique.
[FIG. 3] FIG. 3 is a graph showing the SN-38 concentration in plasma in mice after intravenous injection of SN-38-containing liposomes as a function of the elapsed time.
[FIG. 4] FIG. 4 is a graph showing results of an accelerated preparation stability test for 2 weeks of the SN-38-containing liposome (HSPC:Chol:SA =7:7:5) of the present invention.

### BEST MODE FOR CARRYING OUT THE INVENTION

Hereinafter, the present invention will be described in more detail.

The liposome preparation of the present invention is a liposome preparation as defined in claim 1.

Liposomes are closed vesicles provided in the form of an aqueous dispersion, each of which is composed of a phospholipid bilayer membrane, and has a structure forming a space separated from the outside by a membrane which is generated owing to polarities of hydrophobic groups and hydrophilic groups in lipids. Aqueous phases inside an outside the closed vesicle which are separated by the membrane from each other are referred to as the internal aqueous phase and the external aqueous phase, respectively. The term "liposome preparation" refers to a preparation in which a liposome as a carrier is made to carry a drug. In the present invention, the liposome carries as the drug a slightly water-soluble camptothecin compound. It should be noted that the structure of the liposome of the present invention can be of various forms, and is not particularly limited.

In the present invention, the term "slightly water-soluble camptothecin compound" is used as a generic name for compounds each of which has a camptothecin skeleton and is insoluble or slightly soluble in water. Specific examples thereof include 20(S)-camptothecin represented by the following formula (1) and a slightly water-soluble derivative thereof having a substituent at its 7th position, a derivative of a lipophilic or lipid-soluble nature in particular. Examples of the derivatives include 7-ethyl-10-hydroxycamptothecin (referred to as "SN-38") represented by the following formula (2), 7-(β-trimethylsilyl)ethylcamptothecin (sometimes referred to as "BNP-1350") represented by the following formula (3), and 7-(t-butoxy)iminomethylcamptothecin (sometimes referred to as "Gimatecan" or "ST-1481") represented by the following formula (4).

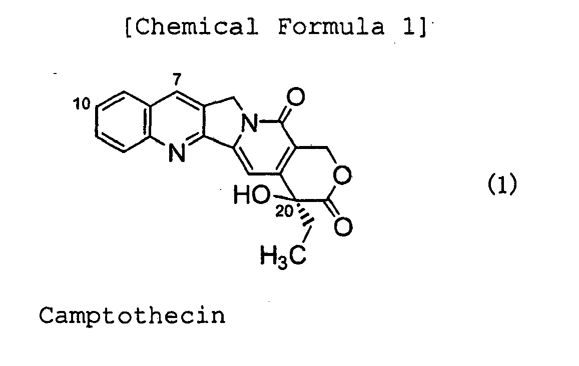

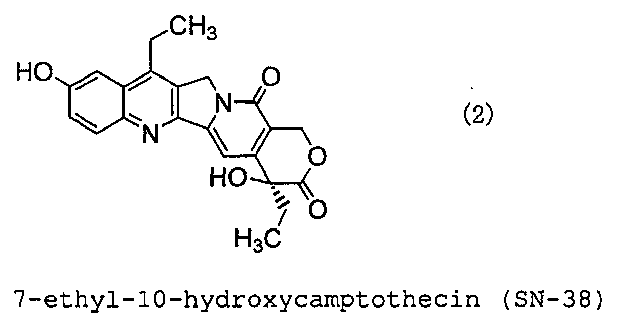

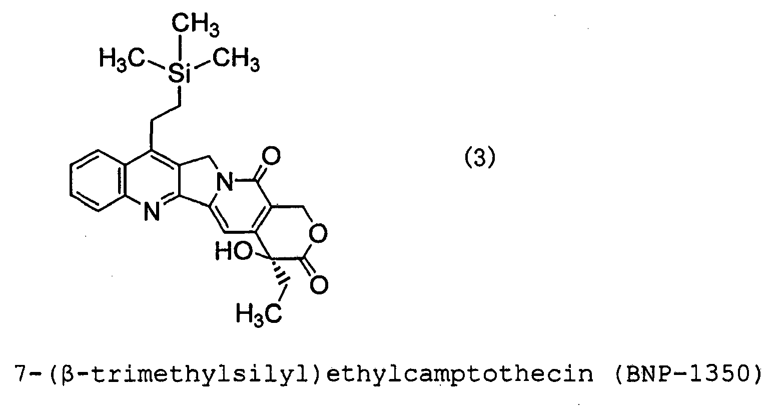

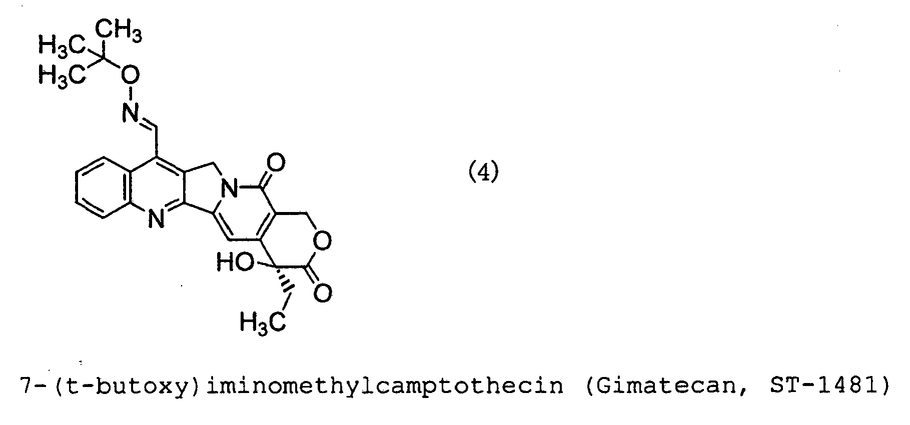

Each of those compounds is known as a drug which exerts an antitumor activity by itself. In addition, the lipophilic or lipid-soluble camptothecin derivatives described in Patent Documents 2 and 3, Non-patent Document 2, and the like can also be examples of the drug of the present invention.

Those compounds can be produced according to the descriptions in the above-mentioned Patent Documents, and some of them are available as commercial products.

Of those, 7-ethyl-10-hydroxycamptothecin (SN-38) is preferable. The compound SN-38 is available from Yakult Honsha Co., Ltd.. Hereinafter, the slightly water-soluble camptothecin compound may simply be referred to as "drug".

The phospholipid that serves as a base material for the membrane of a liposome which carries the above-mentioned drug is a main constituent of a biomembrane. The phospholipid is an amphipathic substance having, in its molecule, hydrophobic groups composed of long chain alkyl groups and hydrophilic groups composed of phosphate groups. Examples of the phospholipid include: natural or synthetic phospholipids such as phosphatidylcholine (sometimes referred to as "lecithin"), phosphatidylglycerol, phosphatidic acid, phosphatidylethanolamine, phosphatidylserine and phosphatidylinositol, as well as sphingophospholipids including sphingomyelin, and cardiolipin, or derivatives thereof; phospholipid derivatives having saccharides bonded thereto (i.e., glycolipids); and hydrogenated phospholipids obtained by adding hydrogen to those phospholipids according to a general method.

Among those, a hydrogenated phospholipid such as hydrogenated soybean phosphatidylcholine (HSPC), sphingomyelin (SM), and the like are preferable.

The liposome preparation of the present invention may contain a single kind of phospholipid or several kinds of phospholipids as a base material for the liposome membrane.

The liposome of the present invention contains as lipid membrane components any of the above-mentioned phospholipids and, especially as an essential component, a saturated fatty acid. Specific examples thereof include myristic acid, pentadecylic acid, palmitic acid, heptadecylic acid, stearic acid, and nonadecanoic acid, The saturated fatty acid has an alkyl chain length of 14 to 18. Stearic acid is particularly preferable. A single kind of fatty acid or two or more kinds of fatty acids may be used.

The content of any fatty acid or fatty acids as above is generally 1 to 30 mol% of the total mol of the lipid components (i.e., of the amount of the lipids in all). In order to further ensure the effect of stabilizing and holding the slightly water-soluble camptothecin compound in the membrane, the fatty acid content is preferably 5 mol% or more, more preferably 10 mol% or more, and further preferably 12 mol% or more. The upper limit of the content is preferably 26 mol% or less in consideration of the stability of the liposome membrane.

It should be noted that, in the present specification, the term "lipids in all" means the whole of the lipids incorporated in a liposome, including the following additional lipids when they are incorporated.

The liposome preparation of the present invention may contain additional components to the extent that the object of the present invention is not impaired. For example, the liposome preparation may contain, in addition to the above-mentioned phospholipid and fatty acid, another membrane component which does not affect the above-mentioned membrane structure and can be incorporated into the liposome. Such a membrane component is not particularly limited, and examples thereof include a lipid which has in its molecule a hydrophobic group composed of a long chain alkyl group and the like and contains no phosphate group in its molecule, such as glyceroglycolipid, sphingoglycolipid and cholesterol, or derivatives thereof, as well as hydrogenated derivatives thereof. One kind or several kinds of additional membrane components may be included.

Cholesterol derivatives are sterols each having a cyclopentanohydrophenanthrene ring, and examples thereof include cholestanol.

Among those, cholesterol has a stabilizing effect on a membrane, and is expected to bring about an effect of controlling the drug release rate of a liposome preparation in plasma depending on the amount of cholesterol. Thus, cholesterol is preferably contained, and it is desired in general that cholesterol be contained in an amount of up to about 50 mol% of the total mol of the lipid components (amount of the lipids in all). The cholesterol content is preferably 20 to 50 mol%, more preferably 30 to 50 mol%, and further preferably 35 to 50 mol%.

In addition, the membrane is modified in order to change properties of the lipids constituting the membrane and impart desired characteristics to the membrane. To be specific, the membrane may include therein a derivative having a modification group linked to the hydrophobic body which is compatible with a lipid membrane, and the hydrophobic body is generally a lipid. The lipid moiety may be any phospholipid, or may be any lipid other than phospholipids, or again, may be any of phospholipids and other lipids, and is not particularly limited. Examples thereof include a phospholipid, a long chain aliphatic alcohol, a sterol, polyoxypropylenealkyl, and a glycerin fatty ester.

Polyethylene glycol (PEG) is used because of having an effect of improving retention in blood. The molecular weight of PEG is not particularly limited, and is 500 to 10,000 daltons in general. Preferably, the molecular weight of PEG is 1,000 to 7,000 daltons, and more preferably 2,000 to 5,000 daltons.

In the present invention, the property of a liposome preparation which is referred to as "retention in blood" is a property of allowing the drug carried by the liposome preparation to stay in blood of a host to whom the liposome preparation has been administered. Once released from a liposome, the drug rapidly disappears from the blood to act on the site to which the drug is exposed. Accordingly, a good retention in blood will enable administration of the drug in a reduced amount.

The problem of the liposomes in a living body, that is, agglomeration due to interaction (i.e., adsorption) between the liposomes and an opsonic protein or a plasma protein in blood, or entrapment in a reticuloendothelial system (RES) of the liver or spleen, can be avoided by membrane modification with the above-mentioned hydrophilic polymer, whereby the stability in blood is increased and highly selective delivery to a target tissue or cell can be achieved. In other words, good retention in blood can be obtained. Accordingly, the liposomes can passively be accumulated in the tissue in which vascular permeability of a tumor tissue is enhanced.

The above-mentioned membrane modification with PEG can be performed by use of a modification agent containing PEG, by addition of PEG through a reaction of a membrane-constituting lipid to which a functional group has been attached with PEG, and the like. The process thereof is not particularly limited, but introduction by using a modification agent containing PEG, such as a phospholipid derivative and/or a cholesterol derivative of PEG, is preferable.

Specific examples thereof include phosphatidylethanolamine ligated with polyethylene glycol (PEG-PE), and cholesterol ligated with polyethylene glycol (Chol-PEG). In addition, when the lipid moiety is composed of a phospholipid, the acyl chain of the phospholipid is preferably a saturated fatty acid. Further, the chain length of the acyl chain is preferably of 14 to 20 carbon atoms, and more preferably 16 to 18 carbon atoms. Specific examples thereof include dipalmitoyl, distearoyl, and palmitoylstearoyl.

Of those, PEG-PE is a general purpose compound and readily available.

The ratio of membrane modification with the above-mentioned hydrophilic polymer chain may generally be 0.1 to 20 mol% of the lipids in all with no hydrophilic polymer chain, and is preferably 0.1 to 5 mol%, and more preferably 0.5 to 5 mol%.

Examples of the charged group include, but not limited to: basic functional groups such as an amino group, an amidino group, and a guanidino group; and acidic functional groups. The charged group can be introduced by using a charged substance having any of those groups.

Examples of the charged substance having a basic functional group include DOTMA which is disclosed in JP 61-161246 A, DOTAP which is disclosed in JP 05-508626 A, Transfectam which is disclosed in JP 02-292246 A, TMAG which is disclosed in JP 04-108391 A, 3,5-dipentadecyloxybenzamidine hydrochloride which is disclosed in WO 97/42166, DOSPA, TfxTM-50, DDAB, DC-CHOL, and DMRIE.

Examples of the charged substance having an acidic functional group include: gangliosides including sialic acid such as ganglioside GM1 and ganglioside GM3; and acidic amino acid-based surfactants such as N-acyl-L-glutamine.

When the above-mentioned charged substance is a substance in which a compound having a basic functional group is bonded to a lipid, the substance is referred to as cationized lipid. The lipid moiety of a cationized lipid is stabilized in the lipid bilayer membrane of a liposome, and the basic functional moiety thereof can be present on membrane surfaces (i.e., outer membrane surface and/or inner membrane surface) of the lipid bilayer of the carrier. Modification of the membrane with a cationized lipid can increase adhesion between the liposome membrane and a cell, and the like.

Examples of the water-soluble polysaccharide include, but not particularly limited to, water-soluble polysaccharides such as glucuronic acid, sialic acid, dextran, pullulan, amylose, amylopectin, chitosan, mannan, cyclodextrin, pectin, and carageenan. Examples of a derivative of the water-soluble polysaccharide include glycolipids.

The ratio of membrane modification with the above-mentioned charged substance or water-soluble polysaccharide can appropriately be set as required.

In the present invention, the amount of the slightly water-soluble camptothecin compound carried in the above-mentioned liposome, that is, the drug/lipid molar ratio of the liposome preparation, is generally 0.1 or less, and preferably 0.001 to 0.03 from viewpoints of stability and encapsulation efficiency of the liposome because the slightly water-soluble camptothecin compound is included in the liposome membrane. In addition, when the slightly water-soluble camptothecin compound/fatty acid molar ratio is smaller than 1.0, the effect of competitive inhibition due to the withdrawal action of a protein in blood becomes clearer.

The drug carried by the liposome, or the drug contained in the liposome preparation, is to be considered as the drug present in the liposome preparation (in dispersion form) as being carried and held by the liposome, that is to say, the drug is essentially not to be contained in the external aqueous phase of the dispersion outside the liposome as being present freely and independently of liposomes. In the liposome preparation of the present invention containing as the drug a slightly water-soluble compound, at least part of the drug is thought to be carried substantially in a lipid membrane by, for example, the incorporation in the membrane. However, in the liposome preparation of the present invention, the state in which the drug is carried is in no way limited as long as the drug is not present freely in the external aqueous phase of the preparation. For example, the drug may be incorporated in the internal aqueous phase independently of the membrane, or incorporated as such and carried by the membrane concurrently.

The liposome preparation of the present invention is characterized in that the preparation is observed as particles with an irregular, nonspherical shape under transmission electron microscopy after freezing in the presence of glycerin. It should be noted that a liposome (preparation) containing no fatty acid is observed by the same method as particles with an original spherical shape.

The lipid bilayer membrane of the liposome may be of a membrane structure of a unilamellar vesicle (e.g., small unilamellar vesicle (SUV) or large unilamellar vesicle (LUV)), or of a multilamellar vesicle (MLV) composed of multiple layers, with MLV being preferable.

The liposome preparation of the present invention shows a nonspherical particulate shape as observed from an electron microphotograph taken by using a freeze-fracture replica technique in the presence of 25% glycerin. The size of a liposome can be measured as the average particle diameter of all the particles by using light scattering and diffraction granulometer. The average particle diameter is preferably 50 to 300 nm, more preferably 70 to 200 nm, and further preferably 90 to 150 nm. In a particular consideration of the efficiency in drug introduction into cells which allows the expression of the antitumor activity in the cells, it is desirable that the average particle diameter is 140 nm or less.

The liposome preparation of the present invention may further contain a pharmaceutically acceptable stabilizer and/or antioxidant depending on the administration route. Examples of the stabilizer include, but not particularly limited to, saccharides such as glycerol and sucrose. Examples of the antioxidant include, but not limited to, ascorbic acid, uric acid, and a tocopherol homologue such as vitamin E. Tocopherol includes 4 isomers, that is, α, P, y, and δ isomers, and any of them can be used in the present invention.

The liposome preparation of the present invention may additionally include a pharmaceutically acceptable additive depending on the administration route. Examples of the additive include water, a physiological saline solution, a pharmaceutically acceptable organic solvent, collagen, polyvinyl alcohol, polyvinylpyrrolidone, a carboxyvinyl polymer, sodium carboxymethylcellulose, sodium polyacrylate, sodium alginate, a water-soluble dextran, sodium carboxymethylstarch, pectin, methylcellulose, ethylcellulose, xanthan gum, gum arabic, casein, gelatin, agar, diglycerine, propylene glycol, polyethylene glycol, vaseline, paraffin, stearyl alcohol, human serum albumin (HSA), mannitol, sorbitol, lactose, PBS, a biodegradable polymer, a serum-free culture medium, a surfactant which is acceptable as a pharmaceutical additive, and a buffer solution of physiological pH that can be tolerated in vivo. The additives to be used are selected from the above-mentioned additives, singly or in combination, appropriately in accordance with the dosage form, but are not limited to the above.

In one aspect of the present invention, the liposome preparation containing any of those additives can be provided as a pharmaceutical composition. The pharmaceutical composition of the present invention can be stored by a general method, for example, in a refrigerator at 0 to 8°C, or at room temperature ranging from 1 to 30°C.

For the liposome preparation as described above, a wide spectrum of known methods of producing a liposome preparation can be used as long as the drug is stably carried therein. It should be noted that steps of dispersion and particle sizing which are described below are each preferably performed at the phase transition temperature of a membrane-forming lipid or a higher temperature.

In the present invention, the method of obtaining a liposome preparation (i.e., dispersion) is not particularly limited. However, it is preferred that a liposome dispersion be prepared from a material mixture of a drug and a lipid preliminarily prepared. The dispersion may be a product obtained by ultrasonically dispersing or mechanically stirring the material mixture. Further, the dispersion may be a jet stream dispersion obtained by using a homogenizer such as Clearmix single (trade name).

When it is desired to reduce the average particle size or to make the particle sizes equal in the present invention, a method may be adopted after the preparation of the liposome dispersion as above (hereinafter, also referred to as "preliminary dispersion") in which particle sizing is performed while subjecting the liposome preparation (i.e., dispersion) to liquid-liquid shearing by means of a high-pressure homogenizer. A specific example is a high-pressure ejection emulsifying method which involves ejecting the liposome dispersion at a high pressure to allow wet grinding due to collision of high-pressure streams with each other (Terada, Yoshimura, et al., "Liposomes in Life Science", Springer-Verlag Tokyo, 1992: the contents of this document are herein incorporated by reference). A high-pressure ejection emulsifier such as Microfluidizer (trade name; manufactured by Microfluidex Inc.) can be used. In addition to the high-pressure ejection emulsifier, a high-pressure homogenizer such as a jet stream emulsifier including Clearmix Double motion (trade name; manufactured by M technique Corp.) may be used.

As a desired average particle size becomes smaller, for instance, up to 300 nm, up to 200 nm, or even up to 150 nm, it is more desirable that the particle sizing is performed with the above-mentioned high-pressure homogenizer.

When a high-pressure homogenizer is used in the particle sizing of a liposome dispersion, attention should be paid to the following. The slightly water-soluble camptothecin compound has a substantially weak interaction with a lipid membrane, so that a high emulsification energy is required to incorporate the slightly water-soluble camptothecin compound in the lipid membrane without fail and allow the lipid membrane to hold the compound. Therefore, when an emulsification method which can not apply high energy, such as a diaphragm emulsification method using an extruder or the like, an ether injection method, and a reverse phase evaporation method which are general as the particle sizing method for liposomes, is used, an efficient holding of the slightly water-soluble camptothecin compound by the lipid membrane tends to be difficult because precipitation of the drug upon emulsification or the like occurs. In addition, the liposome preparation is desired in general to be a product which has been subjected to the particle sizing treatment. In particular, when a liposome preparation having a small particle diameter or a uniform particle diameter is to be obtained, a high emulsification energy is required. Examples of the method which can apply a high emulsification energy include a high-pressure ejection emulsifying method and a jet stream emulsifying method. An emulsifying and particle sizing method which can apply a high shear force is particularly preferable.

The liposome preparation of the present invention is desired in terms of the potency of the drug to be a product in which the portion of the drug which is not carried by the liposome is not included in the external aqueous phase. Therefore, the liposome preparation is subjected to a step of removing the drug portion which is not carried by the liposome from the liposome preparation, and the step is generally performed after a particle sizing step. To be specific, the liposome preparation is subjected to a purification such as gel filtration, dialysis, membrane separation, or centrifugation, whereby the drug portion which is not carried by the liposome preparation can be removed.

The liposome preparation of the present invention arrives at a target site in a state where the drug is included therein. As a result, the drug included in the liposome preparation can be delivered to the target site. The delivery of the drug to the target site may be incorporation of the drug included in the liposome preparation into the target site, or alternatively, the drug may not be incorporated into the target site but influence the target site or its vicinity.

In the present invention, the term "target site" means a particular site which the drug included in the liposome preparation is released to and acts on, and means specifically cells, tissues or organs, or the inside thereof, for each site. Target sites such as cells, tissues, organs, and the inside thereof can each be an object for the treatment with the drug, and the drug exerts its effect by being released and exposed to the target site. Examples of the target site include, but not limited to, a tumor.

Examples of the tumor to be treated include, but not particularly limited to, solid tumors. Specific examples thereof include esophageal cancer, stomach cancer, large intestine cancer, colon cancer, rectal cancer, pancreatic cancer, liver cancer, pharyngeal cancer, lung cancer, prostatic cancer, bladder cancer, breast cancer, uterus cancer, and ovary cancer. The target site includes tumor cells, tumor tissues, tumor organs, and the inside thereof. Thus, in the present invention, the term "disease" means the above-mentioned tumors, and the drug is expected to exert antitumor effects against those tumors.

In the present invention, the term "expose" means that the drug released outside the liposome gives an action to an outer environment. To be specific, the released drug approaches a target site, and exerts its action, that is, an antitumor effect, by coming into contact with the target site. The drug acts on a target site, so that the drug topically acts on a cell of the target site, which is in a cell cycle in which DNA synthesis is carried out, and exerts the expected effect. In order to cause the drug to exert such an effect, the ratio of releasing a drug from a liposome preparation and the retention in blood of the liposome preparation should be balanced.

In the present invention, the term "release" means that a drug included in a liposome preparation is released from a liposome owing to passage of the drug through a lipid membrane of the liposome or a change in part of a structure of the lipid membrane.

The drug included in the liposome preparation of the present invention exerts a strong antitumor activity in plasma when exposed to a target site at a high concentration for a long period of time, so it is important to control the release thereof.

The term "releasing ratio" refers to a ratio (i.e., weight ratio or molar ratio) of a portion of a drug which is released from a liposome carrying the drug and a portion of the drug which is left carried in the liposome. The term "low releasing ratio" means that the amount of the drug released from the liposome per unit time is small.

The releasing ratio can be calculated by precipitating the carrier of the present invention by centrifugation, and measuring the amounts of the drug present in a supernatant (i.e., plasma) and the carrier.

As shown in Examples to be described below, the liposome preparation of the present invention was found to be low in drug releasing ratio within a predetermined period of time.

The liposome preparation of the present invention can maintain a drug concentration in plasma at a high level. Conventional liposome preparations rapidly disappear from blood, so exposure time at a target site is short, and thus it is difficult for the conventional liposome preparations to exert sufficient effects. In addition, the rapid disappearance from blood is not preferable because it causes metabolic organs such as liver and spleen to be exposed to the drug at a high concentration, resulting in side effects at the sites. The liposome preparation of the present invention can maintain a drug concentration in plasma at a high level. Therefore, the liposome preparation of the present invention is preferable because the dose of the drug can be reduced, the drug can be exposed to a target site for a long period of time, and the side effects can be thus reduced.

In the present invention, the drug concentration in plasma 1 hour after administration is 10% or more, and preferably 15% or more of the initial value. The term "initial value" refers to the theoretical concentration immediately after the administration of the liposome preparation of the present invention, and is generally calculated from a total plasma amount calculated from the weight of a host, assuming that the total plasma is diluted with the liquid in an administration amount. In addition, the concentration in plasma in each time point can be represented as a ratio to the initial value, and generally represented as "% of dose".

The present invention is used for exposing a drug to a desired target site for a long period of time. Therefore, in the present invention, a liposome preparation which carries an effective amount of drug is administered to a host to prevent and/or treat a disease of the host, whereby the effective amount of drug can be released in the host and exposed to the target site for a long period of time at a high concentration, so the liposome preparation can be parenterally administered to the host (i.e., patient) in a systemic or topical manner. Examples of the host to which the liposome preparation may be administered include mammals, and preferably human, monkey, mouse, and domestic animals.

The route of the parenteral administration can be selected from, for example, intravenous injection (IV) such as instillation, intramuscular injection, intraperitoneal injection and subcutaneous injection, and the administration method can appropriately be selected depending on the age and condition of a patient. The carrier of the present invention is administered to a patient suffering from a disease, in a sufficient amount to treat the condition of the disease in the patient or at least partly prevent the condition. For example, the effective dose of a drug to be encapsulated in the carrier is selected in a range of 0.01 mg to 100 mg/kg body weight/day. However, the dose of the carrier of the present invention is not limited thereto. The administration may be started after the onset of a disease, or may prophylactically be performed for alleviating the condition of a disease in a patient at the time of its onset when the disease is expected to occur. In addition, the administration period may appropriately be selected depending on the age and condition of a patient.

A specific administration method includes administration of a pharmaceutical composition by means of a syringe or instillation. The pharmaceutical composition can also be administered as follows: A catheter is inserted into the body of a patient or a host, for example, into a lumen like a blood vessel, so that the proximal end of the catheter approaches the vicinity of a target site; and the pharmaceutical composition is administered to a desired target site or its vicinity, or from a site where a blood flow to the desired target site is expected.

### EXAMPLES

Next, the present invention will be described in further detail by way of examples. However, the following examples are merely illustrative of the present invention, and the present invention is not limited to these examples.

### (Examples 1 to 3)

### (1) Preparation of material mixture

Hydrogenated soybean phosphatidylcholine (manufactured by Lipoid GmbH) (hereinafter, referred to as "HSPC"), cholesterol (manufactured by Solvay S. A.) (hereinafter, referred to as "Chol"), stearic acid (manufactured by NOF CORPORATION) (hereinafter, referred to as "SA"), and 7-ethyl-10-hydroxycamptothecin (SN-38, manufactured by Yakult Honsha Co., Ltd.) (hereinafter, referred to as "SN-38") were weighed so as to have predetermined molar ratios as shown in Table 1, and dissolved in 200 mL of t-butanol (manufactured by Kanto Chemical Co., Inc.) which had been heated to 60°C. After that, the solution was cooled on ice and lyophilized.

### (2) Dispersion

By addition of 200 mL of physiological saline, the resulting dried product was preliminarily dispersed using CLEARMIX SINGLE (manufactured by M Technique Co., Ltd.) while heating at 68°C. After that, a microfluidizer (manufactured by Microfluidex Inc.) was used to control the particle diameter.

### (3) Introduction of PEG-PE

To 50 mL of the resulting liposome dispersion was added 11.32 mL of a physiological saline solution containing polyethylene glycol (having a molecular weight of 5,000) - phosphatidylethanolamine (manufactured by Genzyme Corporation) (hereinafter, referred to as PEG-PE) at a concentration of 36.74 mg/mL so that the ratio of PEG-PE introduction to the lipids in all was about 2 mol%. The mixture was heated at 60°C for 30 minutes and then cooled on ice. The external aqueous phase of the liposome dispersion after the introduction of PEG-PE was substituted by using a gel column which had been sufficiently substituted by physiological saline, to thereby remove the non-encapsulated drug portion.

Table 1 shows the membrane compositions, the amounts of a carried drug (drug/lipid ratio and drug concentrations), and the particle diameters of the thus obtained SN-38 liposome preparations.

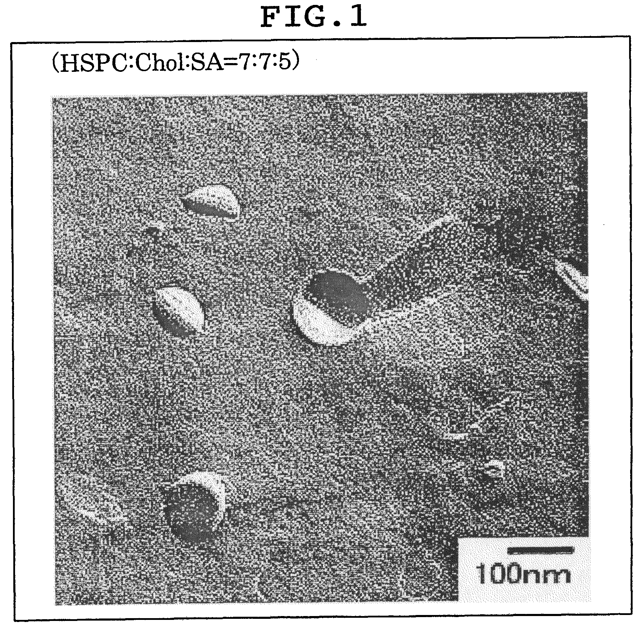

In addition, Fig. 1 shows an electron micrograph of a liposome preparation obtained in Example 3 which was taken by a freeze-fracture replication technique in the presence of 25% glycerin. It was observed that the liposome preparation had a nonspherical shape and was heteromorphic.

### (Comparative Example 1)

A liposome preparation was prepared in the same manner as in Example 1 except that stearic acid (SA) was not used in the above-mentioned step (1). In other words, HSPC, Chol, and SN-38 weighed as shown in Table 1 were dissolved in 200 mL of t-butanol (manufactured by Kanto Chemical Co., Inc.) at 60°C, and the solution was cooled on ice and lyophilized. Then, by addition of 200 mL of physiological saline, the resulting product was preliminarily dispersed using CLEARMIX while heating at 68°C. After that, a microfluidizer was used to control the particle diameter.

To 50 mL of the resulting liposome dispersion was added 11.32 mL of a physiological saline solution containing 36.74 mg/ml of PEG-PE. The mixture was heated at 60°C for 30 minutes and then cooled on ice. The external aqueous phase of the liposome dispersion after the introduction of PEG-PE was substituted by using a gel column which had been sufficiently substituted by physiological saline, to thereby remove the non-encapsulated drug portion.

Table 1 shows the membrane composition, the amount of a carried drug (drug/lipid molar ratio and drug concentration), and the particle diameter of the thus obtained SN-38 liposome preparation.

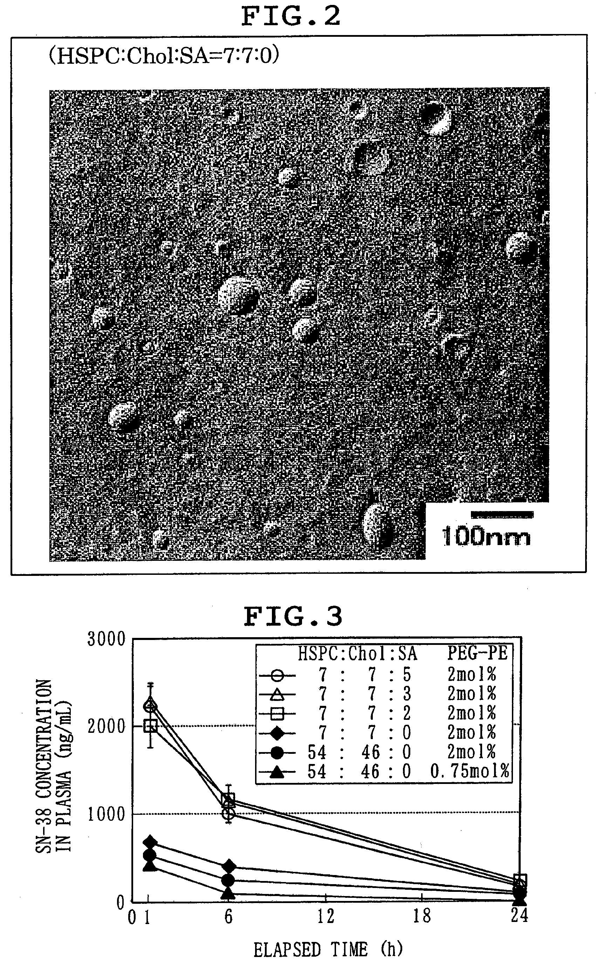

Fig. 2 shows an electron micrograph of a liposome preparation obtained in Comparative Example 1 which was taken by a freeze-fracture replication technique in the presence of 25% glycerin. It was observed that the liposome preparation had a spherical shape.

### (Comparative Examples 2 and 3)

Liposome preparations having different membrane compositions (lipid ratios and PEG-PE introduction ratios) and different amounts of carried drug were prepared without using stearic acid (SA) as in Comparative Example 1. In other words, HSPC, Chol, and SN-38 weighed as shown in Table 1 were dissolved in 200 mL of t-butanol (manufactured by Kanto Chemical Co., Inc.) at 60°C, and the solution was cooled on ice and lyophilized. Then, by addition of 300 mL of physiological saline, the resulting product was preliminarily dispersed using CLEARMIX while heating at 68°C. After that, a microfluidizer was used to control the particle diameter.

To 50 mL each of the resulting liposome dispersions were added 2.916 mL (PEG-PE introduction ratio: about 0.75 mol%) and 7.776 mL (PEG-PE introduction ratio: about 2 mol%) of a physiological saline solution containing 36.74 mg/ml of PEG-PE, respectively. Each of the mixtures was heated at 60°C for 30 minutes and then cooled on ice. The external aqueous phase of the liposome dispersion after the introduction of PEG-PE was substituted by using a gel column which had been sufficiently substituted by physiological saline, to thereby remove the non-encapsulated drug portion.

Table 1 shows the membrane compositions, the amounts of a carried drug (drug/lipid ratios and drug concentrations), and the particle diameters of the thus obtained SN-38 liposome preparations.

[Table 1]

**Table 1**

| | | | | Example 1 | Example 2 | Example 3 | Comparative Example 1 | Comparative Example 2 | Comparative Example 3 |
|---|---|---|---|---|---|---|---|---|---|
| | | Weight/g | HSPC | 4.835 | 4.557 | 4.078 | 5.534 | 6.000 | |
| | | | Chol | 2.366 | 2.231 | 1.996 | 2.708 | 2.504 | |
| | | | SA | 0.504 | 0.703 | 1.049 | - | | |
| | | | SN-38 | 0.040 | | | | | |
| Membrane | | HSPC:Chol:SA molar ratio | | 7:7:2 | 7:7:3 | 7:7:5 | 7:7:0 | 54:46:0 | |
| | | SA content (mol%) | | 12.25 | 17.25 | 25.78 | 0 | 0 | 0 |
| | | PEG-PE introduction ratio (mol%) | | 1.96 | | | | | 0.744 |
| Preparation | | Drug*/lipid molar ratio | | 0.0069 | | | | 0.0098 | 0.0099 |
| | | Drug* concentration | (µg/mL) | 84 | 85 | 73 | 68 | 68 | 72 |
| | | Particle diameter | (nm) | 87.4 | 100 | 122 | 114 | 91.6 | 93.7 |
| Drug* concentration in plasma (ng/mL) | | | | | | | | | |
| | After administration | | 1 hour | 1994 | 2278 | 2222 | 669 | 535 | 439 |
| | | | 6 hours | 1152 | 1133 | 988 | 390 | 265 | 113 |
| | | | 24 hours | 214 | 185 | 155 | 81 | 66 | 10 |
| % of dose | | | | | | | | | |
| | After administration | | 1 hour | 19.17 | 21.90 | 21.37 | 6.43 | 5.14 | 4.22 |
| | | | 6 hours | 11.08 | 10.89 | 9.50 | 3.75 | 2.55 | 1.09 |
| | | | 24 hours | 2.06 | 1.78 | 1.49 | 0.78 | 0.63 | 0.10 |

| | | | | | | | | | |
|---|---|---|---|---|---|---|---|---|---|
| *Drug: SN-38 | | | | | | | | | |

In the table, the term "lipid" refers to a lipid mixture (HSPC+Chol+SA+PE) including SA and PE portion of PEG-PE.

The SA content refers to the amount of SA with respect to the total amount of the lipid mixture (i.e., total amount of lipids), which is represented by mol%.

The PEG-PE introduction ratio refers to the ratio of the PEG-PE amount to the total amount of the lipid mixture, which is represented by mol%.

The amount of a carried drug in a preparation, that is, a drug/lipid molar ratio, refers to the molar ratio of SN-38 to the lipid mixture, and the drug concentration refers to the amount of SN-38 (µg) per mL of a liposome preparation.

The drug concentration in a preparation or plasma was obtained by measuring fluorescence intensity of SN-38 by using a spectrofluorometer. To be specific, a sample was diluted 10 folds with methanol, and to 20 µL of the diluted sample was added 2 mL of methanol. Fluorescence of the resulting solution was measured at an excitation wavelength of 380 nm and a fluorescence wavelength of 430 nm.

The particle diameter is a value obtained by subjecting a liposome preparation to measurement with a light diffraction scattering granulometer (Coulter LS230, manufactured by Beckman Coulter, Inc.), and means the average particle diameter.

### (Test Example 1) Retention in blood

The SN-38 liposome preparation obtained as described above was injected to each of tail veins of mice (BALB/c, female, 5-weeks-old, CLEA Japan Inc.) in an amount of 0.4 mg/kg as the SN-38 amount. Blood was collected 1, 6, and 24 hours after the injection, and subjected to centrifugation (3,000 rpm, 10 minutes, 4°C), to thereby collect plasma. The plasma was stored in a refrigerator until the subsequent measurement of fluorescence intensity.

The SN-38 concentration in each plasma was measured. Results are shown in Table 1 and Fig. 3.

As shown in Fig. 3, the liposome preparations of the present invention which contained SA in their membrane components indicated the SN-38 concentrations in plasma about three to four times at 1 hour and about two to three times at 24 hours as large as the liposome preparations containing no SA. In addition, when the concentration was represented by "% of dose", the liposome preparations of the present invention which contained SA had SN-38 concentrations in plasma of about 19 to 21% at 1 hour, whereas the liposome preparations containing no SA had SN concentrations in plasma of about 4 to 6%. Thus, it was confirmed that the liposome preparation containing SA according to the present invention is capable of maintaining the SN-38 concentration in plasma at high levels for a long period of time.

In addition, an improvement in the retention in blood owing to the increase in the PEG-PE introduction ratio was observed when the liposome preparation containing SN-38 was used. However, the retention in blood remained at a low level with the introduction of PEG-PE alone.

### (Example 4)

Procedures in Example 3 were repeated for preparing another lot of Example 3. Table 2 shows the weights, and the membrane composition, the amount of a carried drug (drug/lipid molar ratio and drug concentration) and the particle diameter of the thus obtained SN-38 liposome preparation.

[Table 2]

**Table 2**

| | Weight/g | | | | Mixed lipid ratio | PEG-PE introduction ratio | SN-38/ lipid | SN-38 concentration | Particle diameter |
|---|---|---|---|---|---|---|---|---|---|
| | HSPC | Chol | SA | SN-38 | HSPC:Chol: SN-38 | mol% | Molar ratio | µg/mL | nm |
| Example 4 | 4.078 | 1.996 | 1.049 | 0.040 | 7:7:5 | 1.96 | 0.0069 | 33.6 | 99.7 |

### (Test Example 2) Storage stability

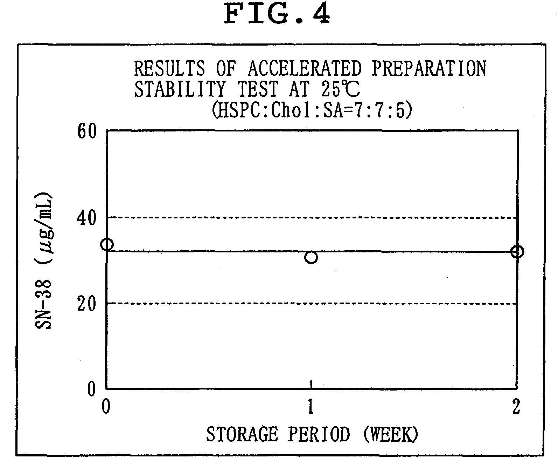

The thus obtained SN-38 liposome preparation (HSPC:Chol:SA = 7:7:5) was subjected to an accelerated preparation stability test at 25°C, and the SN-38 content in the preparation was measured with a spectrofluorometer. Fig. 4 shows the results thereof.

As shown in Fig. 4, the SN-38 content did not decrease for 2 weeks. Thus, it was confirmed that the SN-38 liposome preparation of the present invention is excellent in preparation stability.

## Claims

1. A liposome preparation, including as lipid components for forming a membrane a phospholipid and a saturated fatty acid having an alkyl chain length of 14 to 18, and containing as a drug a slightly water-soluble camptothecin compound wherein said membrane has a modification moiety which comprises a polyethylene glycol chain.

2. The liposome preparation according to claim 1, wherein said slightly water-soluble camptothecin compound comprises 20(S)-camptothecin, 7-ethyl-10-hydroxycamptothecin (SN-38), 7-(ß-trimethylsilyl)ethylcamptothecin, or 7-(t-butoxy)iminomethylcamptothecin.

3. The liposome preparation according to claim 1 or 2, wherein said phospholipid comprises a hydrogenated phospholipid.

4. The liposome preparation according to any one of claims 1 to 3, further comprising as said lipid components at least one other lipid in addition to the phospholipid and the fatty acid.

5. The liposome preparation according to claim 4, in which the other lipid comprises cholesterol.

6. The liposome preparation according to any one of claims 1 to 5, wherein the liposome preparation has a drug/lipid molar ratio of 0.001 to 0.1 or less.

7. The liposome preparation according to any one of claims 1 to 6, wherein said fatty acid is included in a ratio of 1 to 30 mol% of the total mol of the lipid components.

8. The liposome preparation according to any one of claims 1 to 7, wherein said liposome preparation has an average particle diameter of 50 to 300 nm.

9. The liposome preparation according to any one of claims 1 to 8, wherein said liposome preparation includes a liposome preparation which is producible by stirring the lipid components and the slightly water-soluble camptothecin compound to prepare a liposome dispersion, and subjecting the liposome dispersion to a particle sizing treatment by causing high-pressure streams of the liposome dispersion to collide with each other.

10. A pharmaceutical composition, including the liposome preparation according to any one of claims 1 to 9.

## Patentansprüche

1. Liposomzubereitung, die ein Phospholipid und eine gesättigte Fettsäure mit einer Alkylkettenlänge von 14 bis 18 als Lipidbestandteile für die Bildung einer Membran beinhaltet, und die eine schwach wasserlösliche Camptothecinverbindung als einen Wirkstoff enthält, wobei die Membran eine Modifikationsgruppe aufweist, welche eine Polyethylengglycolkette umfasst.

2. Liposomzubereitung nach Anspruch 1, wobei die schwach wasserlösliche Camptothecinverbindung 20(S)-Camptothecin, 7-Ethyl-10-hydroxycamptothecin (SN-38), 7-(ß-Trimethylsilyl)ethylcamptothecin oder 7-(t-Butoxy)iminomethylcamptothecin umfasst.

3. Liposomzubereitung nach Anspruch 1 oder 2, wobei das Phospholipid ein hydriertes Phospholipid umfasst.

4. Liposomzubereitung nach einem der Ansprüche 1 bis 3, das zusätzlich zu dem Phospholipid und der Fettsäure ferner wenigstens ein anderes Lipid als die Lipidbestandteile umfasst.

5. Liposomzubereitung nach Anspruch 4, in welcher das andere Lipid Cholesterol umfasst.

6. Liposomzubereitung nach einem der Ansprüche 1 bis 5, wobei die Liposomzubereitung ein Wirkstoff-/Lipid-Molverhältnis von 0,001 bis 0,1 oder weniger aufweist.

7. Liposomzubereitung nach einem der Ansprüche 1 bis 6, wobei die Fettsäure in einem Verhältnis von 1 bis 30 Mol-% zu dem gesamten Mol der Lipidbestandteile beinhaltet ist.

8. Liposomzubereitung nach einem der Ansprüche 1 bis 7, wobei die Liposomzubereitung einen durchschnittlichen Teilchendurchmesser von 50 bis 300 nm aufweist.

9. Liposomzubereitung nach einem der Ansprüche 1 bis 8, wobei die Liposomzubereitung eine Liposomzubereitung beinhaltet, welche herstellbar ist durch Rühren der Lipidbestandteile und der schwach wasserlöslichen Camptothecinverbindung, um eine Liposomdispersion zuzubereiten, und Unterziehen der Liposomdispersion mit einer Teilchengrößenbehandlung durch Bewirken, dass Hochdruckströme der Liposomdispersion miteinander kollidieren.

10. Pharmazeutische Zusammensetzung, die die Liposomzubereitung nach einem der Ansprüche 1 bis 9 beinhaltet.

## Revendications

1. Préparation de liposomes, comprenant comme constituants lipidiques pour former une membrane, un phospholipide et un acide gras saturé ayant une longueur de chaîne alkyle de 14 à 18, et contenant en tant que médicament un composé camptothécine légèrement soluble dans l'eau, dans laquelle ladite membrane a une fraction de modification qui comprend une chaîne polyéthylène glycol.

2. Préparation de liposomes selon la revendication 1, dans laquelle ledit composé camptothécine légèrement soluble dans l'eau comprend la 20(S)-camptothécine, la 7-éthyl-10-hydroxycamptothécine (SN-38), la 7-(β-triméthylsilyl)éthylcamptothécine ou la 7-(t-butoxy)iminométhylcamptothécine.

3. Préparation de liposomes selon la revendication 1 ou 2, dans laquelle ledit phospholipide comprend un phospholipide hydrogéné.

4. Préparation de liposomes selon l'une quelconque des revendications 1 à 3, comprenant en outre en tant que lesdits constituants lipidiques au moins un autre lipide, en plus du phospholipide et de l'acide gras.

5. Préparation de liposomes selon la revendication 4, dans laquelle l'autre lipide comprend le cholestérol.

6. Préparation de liposomes selon l'une quelconque des revendications 1 à 5, dans laquelle la préparation de liposomes a un rapport molaire de médicament/lipides de 0,001 à 0,1 ou moins.

7. Préparation de liposomes selon l'une quelconque des revendications 1 à 6, dans laquelle ledit acide gras est inclus dans un rapport de 1 à 30 % en moles du total de moles des constituants lipidiques.

8. Préparation de liposomes selon l'une quelconque des revendications 1 à 7, dans laquelle ladite préparation de liposomes a un diamètre moyen de particule de 50 à 300 nm.

9. Préparation de liposomes selon l'une quelconque des revendications 1 à 8, dans laquelle ladite préparation de liposomes comprend une préparation de liposomes qui peut être produite en agitant les constituants lipidiques et le composé camptothécine légèrement soluble dans l'eau pour préparer une dispersion de liposomes, et en soumettant la dispersion de liposomes à un traitement de calibrage des particules en amenant des courants à haute pression de la dispersion de liposome à entrer en collision les uns avec les autres.

10. Composition pharmaceutique, comprenant la préparation de liposomes selon l'une quelconque des revendications 1 à 9.
